# FASCICULE DE BREVET EUROPEEN

(11) **EP 1 469 814 B1**
(45) Date de publication et mention de la délivrance du brevet: **26.09.2007**
(21) Numéro de dépôt: 02788039.2
(22) Date de dépôt: 06.11.2002
(51) Int. Cl.: A61K 8/81, A61Q 1/06

(54) **COMPOSITION COMPRENANT UN MELANGE DE POLYMERES SEMI-CRISTALLINS ET UNE HUILE VOLATILE**
ZUSAMMENSETZUNG DIE EINE MISCHUNG AUS TEILKRISTALLINEN POLYMEREN UND FLÜCHTIGES ÖL ENTHÄLT
COMPOSITION COMPRISING A MIXTURE OF SEMI-CRYSTALLINE POLYMERS AND A VOLATILE OIL

(30) Priorité: 24.01.2002 FR 0200885; 25.02.2002 FR 0202358
(43) Date de publication de la demande: 27.10.2004
(73) Titulaire: L'ORÉAL, 75008 Paris (FR)
(72) Inventeur: FERRARI, Véronique, F-94700 Maisons-Alfort (FR)
(74) Mandataire: Le Coupanec, Pascale A.M.P.
(86) Numéro de dépôt international: PCT/FR2002/003801
(87) Numéro de publication internationale: WO 2003/061612

(56) Documents cités:
- EP-A- 1 034 776
- EP-A- 1 262 164
- WO-A-01/19333
- US-A- 5 302 380
- US-A- 5 519 063

## Description

La présente invention se rapporte à une composition, notamment cosmétique, contenant un mélange de deux polymères semi-cristallins et une huile volatile, se présentant notamment sous forme d'un stick et dont l'application conduit à un dépôt brillant et confortable, qui ne transfert pas.

Des compositions cosmétiques dont la phase grasse est gélifiée par des polymères semi-cristallins ont été décrites la demande FR 0106047 non encore publiée. Ces compositions à appliquer sur les lèvres, lorsqu'elles sont appliquées sur la peau, présentent toutefois l'inconvénient de transférer, c'est-à-dire de se déposer au moins en partie, en laissant une trace, sur certains supports avec lesquels elles peuvent être mises en contact, et notamment un verre, un vêtement ou la peau. Il s'en suit une persistance médiocre du film sur les lèvres d'où la nécessité de renouveler régulièrement l'application de la composition de rouge à lèvres.

Parallèlement, les utilisatrices de rouge à lèvres non-transfert se plaignent souvent du manque de confort et de la brillance de ce type de formules qui par ailleurs présentent une très bonne tenue.

Améliorer les performances des rouge à lèvres non-transfert est une problématique que nombre de cosméticiens ont essayé de résoudre. On peut ainsi noter SHISEIDO (JP-A-61-65809), PROCTER et GAMBLE (WO-A-96/40004) ou REVLON (US-A-5837223).

Dans la demande FR-A-2 804 018, L'OREAL a décrit l'association de solvant volatil avec des polymères à hétéroataome du type polyamide. Ces compositions, qui conduisent à des rouge à lèvres dont le dépôt est non-transfert et brillant, ont cependant l'inconvénient de présenter un caractère collant qui peut être rédhibitoire et jugé comme inconfortable.

La présente invention a pour but de pallier ces inconvénients et propose une composition qui permet d'obtenir un film de très bonne tenue, qui ne transfère pas, et dont la brillance est améliorée par rapport aux compositions non-transfert de l'art antérieur.

De façon surprenante, le demandeur a trouvé que l'ajout d'une huile volatile dans une composition contenant un mélange de deux polymères semi-cristallins permet d'obtenir une composition non-transfert, dont la brillance est améliorée.

L'invention s'applique en particulier aux produits de maquillage des lèvres mais aussi des yeux comme les eye-liners en particulier sous forme de crayon et les mascaras notamment sous forme de pain, ou de la peau, comme les fonds de teint.

De façon plus précise, l'invention a pour objet une composition de maquillage comprenant
a) au moins une phase grasse liquide structurée par au moins un mélange d'un polymère semi-cristallin à structure organique dont la température de fusion est supérieure ou égale à 30°C, et inférieur à 50°C et d'un polymère semi-cristallin dont la température de fusion est au moins égale à 50°C, b) une matière colorante et c) une huile volatile, la phase grasse liquide, la matière colorante, l'huile volatile et le polymère formant un milieu physiologiquement acceptable.

La composition de l'invention peut se présenter sous forme de pâte, de solide, de crème. Elle peut être une émulsion huile-dans-eau ou eau-dans-huile, un gel anhydre, solide ou souple. De préférence, elle se présente sous forme anhydre, et plus spécialement sous forme de gel anhydre, notamment coulée en stick ou en coupelle.

Par "polymère semi-cristallin", on entend au sens de l'invention, des polymères comportant une partie cristallisable et une partie amorphe dans le squelette et présentant une température de changement de phase réversible du premier ordre, en particulier de fusion (transition solide-liquide). La partie cristallisable est soit une chaîne latérale (ou chaîne pendante), soit une séquence dans le squelette.

Par "polymères", on entend au sens de l'invention des composés comportant au moins 2 motifs de répétition, de préférence au moins 3 motifs de répétition et plus spécialement au moins 10 motifs répétitifs.

Lorsque la partie cristallisable est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.

Par "composé organique" ou "à structure organique", on entend des composés contenant des atomes de carbone et des atomes d'hydrogène et éventuellement des hétéroatomes comme S, O, N, P seuls ou en association.

### Les polymères semi-cristallins

De façon avantageuse, les polymères semi-cristallins de la composition de l'invention comprennent une masse moléculaire moyenne en poids Mp allant de 5 000 à 1 000 000, de préférence de 10 000 à 800 000, préférentiellement de 15 000 à 500 000.

Le ou les polymères semi-cristallins selon l'invention servant d'agent structurant sont des solides à température ambiante (25°C) et pression atmosphérique (760 mm de Hg), dont la température de fusion est supérieure ou égale à 30°C. Les valeurs de point de fusion correspondent au point de fusion mesuré à l'aide d'un calorimètre à balayage différentiel (D. S. C), tel que le calorimètre vendu sous la dénomination DSC 30 par la société METTLER, avec une montée en température de 5 ou 10°C par minute. (Le point de fusion considéré est le point correspondant à la température du pic le plus endotherme du thermogramme).

Les polymères semi-cristallins selon l'invention ont de préférence une température de fusion supérieure à la température du support kératinique destiné à recevoir ladite composition, en particulier la peau ou les lèvres.

Les polymères semi-cristallins selon l'invention sont capables de structurer seuls ou en mélange, la composition sans ajout de tensioactif particulier, ni de charge, ni de cire.

Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

De préférence, le squelette polymérique des polymères semi-cristallins est soluble dans la phase grasse liquide.

De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique.

Les polymères semi-cristallins utilisables dans l'invention sont en particulier :
- les copolymères séquencés de polyoléfines à cristallisation contrôlée, dont les monomères sont décrits dans EP-A-0 951 897.
- les polycondensats et notamment de type polyester, aliphatique ou aromatique ou aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s), tels que décrits dans le document WO-A-01/19333,
- et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans les documents US-A-5 156 911 et WO-A-01/19333.
- Ce sont des homopolymères ou copolymères comportant de 50 à 100 % en poids de motifs résultant de la polymérisation de un ou plusieurs monomères porteurs de chaîne latérale hydrophobe cristallisable.
- Ces homo- ou co-polymères sont de toute nature du moment qu'ils présentent les conditions indiquées ci-après avec en particulier la caractéristique d'être solubles ou dispersables dans la phase grasse liquide, par chauffage au-dessus de leur température de fusion Pf. Ils peuvent résulter :
   - de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
   - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X : 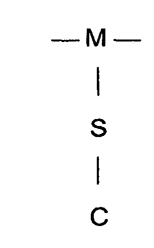 avec M représentant un atome du squelette polymérique
   S représentant un espaceur
   C représentant un groupe cristallisable

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. de préférence en C₁₆-C₂₂ .Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :

### α) de Y qui est un monomère polaire ou non polaire ou un mélange des deux :

- Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
- Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène, un macromonomère du type polyorganosiloxane à insaturation vinylique.

Par "alkyle", on entend au sens au sens de l'invention un groupement saturé notamment en C₈ à C₂₄, sauf mention exprès.
β) de Z qui est un monomère polaire ou un mélange de monomères polaires. Dans ce cas, Z a la même définition que le "Y polaire" défini ci-dessus.
   De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Il s'agit encore de polymères solubles ou dispersables dans la phase grasse liquide par chauffage au-dessus de leur point de fusion Pf. Ces polymères sont notamment des copolymères séquencés constitués d'au moins deux séquences de nature chimique différente dont l'une est cristallisable.
- On peut utiliser les polymères définis dans le brevet US-A-5 156 911,
- Les copolymères séquencés d'oléfine ou de cyclooléfine à chaîne cristallisable comme ceux issus de la polymérisation séquencée de :
   - cyclobutène, cyclohexène, cyclooctène, norbomène (c'est-à-dire bicyclo(2,2,1)heptène-2), 5-méthylnorbomène, 5-éthylnorbomène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   - avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   et en particulier les copoly(éthylène/norbornène) blocs et les terpolymères (éthylène/propylène/éthylidène-norbomène), blocs. On peut aussi utiliser ceux résultants de la copolymérisation séquencée d'au moins 2 α-oléfines en C₂-C₁₆ et mieux en C₂-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   - Séquence cristallisable par nature : a) polyester comme les poly(alkylène téréphtalate), b) polyoléfine comme les polyéthylènes ou polypropylènes.
   - Séquence amorphe et lipophile comme : les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(ε-caprolactone)-b-poly(butadiène), utilisés de préférence hydrogénés, tels que ceux décrits dans l'article D6 "Melting behavior of poly(-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article D7 "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles D8 "Morphology of semi-crystalline block copolymers of ethylene-(ethylene-alt-propylene)" de P. Rangarajan et al., Macromolecules, 26, 4640-4645 (1993) et D9 "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général D10 "Cristallization in block copolymers" de I.W. Hamley, Advances in Polymer Science, vol 148, 113-137 (1999).

Les polymères semi-cristallins de la composition de l'invention peuvent être ou non réticulés en partie du moment où le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

A titre d'exemple particulier de polymère semi-cristallins structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

### Mélange de polymère semi-cristallin à haut point de fusion et de polymère semi-cristallin à bas point de fusion

Dans la suite de la description, le ou les polymères semi-cristallins ayant une température de fusion Pf₂ supérieure ou égale à 30°C et inférieure à 50°C seront dénommés "polymères à bas point de fusion" et le ou les composés cristallins ou semi-cristallins ayant une température de fusion Pf₁ supérieure ou égale à 50°C seront dénommés "composés à haut point de fusion". Selon l'invention, le point de fusion peut être mesuré notamment par toute méthode connue et en particulier avec un calorimètre à balayage différentiel (D.S.C).

La composition contient un mélange d'un polymère choisi parmi les polymères à bas point de fusion ayant une température de fusion supérieure ou égale à 30°C et inférieure à 50°C, et d'un polymère choisi parmi les polymères à haut point de fusion ayant une température de fusion au moins égale à 50°C.

Selon l'invention le ou les composés semi-cristallins à haut point de fusion sont avantageusement des polymères ayant une température de fusion Pf₁ telle que 50°C ≤ Pf₁ ≤ 150°C, mieux 55°C ≤ Pf₁ ≤ 150°C, et de préférence 60°C ≤ Pf₁ ≤ 130°C et les polymères à bas point de fusion ont une température de fusion Pf₂ telle que 30°C ≤ Pf₂ < 50°C, et mieux 35°C ≤ Pf₂ ≤ 45°C. Cette température de fusion est une température de changement d'état du premier ordre.

De façon générale, les polymères à bas point de fusion présentent une température de fusion Pf₂ au moins égale à la température du support kératinique devant recevoir la composition selon l'invention.

Comme composé à haut point de fusion utilisable dans l'invention, on peut citer les cires à haut point de fusion comme certaines cires de polyéthylène telle que l'Epolène N-14 vendu par Eastman Chemical Cie, les cires de Carnauba et certaines cires microcristallines comme celles vendues par Tisco sous la marque "Tisco wax 88", ainsi que des polymères semi-cristallins à haut point de fusion. De préférence, le composé à haut point de fusion est un second polymère semi-cristallin solide organique à haut point de fusion. Il est toutefois possible d'utiliser comme composé à haut point de fusion des polymères cristallins, solides à température ambiante et ayant une température de fusion supérieure à 50°C des polymères statistiques comportant une cristallisation contrôlée, tels que décrits dans le document EP-A-0 951 897 et plus spécialement les produits commerciaux Engage 8 401 et Engage 8 402 de Dupont de Nemours, respectivement de température de fusion de 51°C et 64°C et qui sont des bipolymères statistiques éthylène/1-octène.
i) Les polymères semi-cristallins dont le point de fusion est inférieur à 50°C sont notamment ceux décrits dans les exemples 3, 4, 5, 7, 9 du brevet US-A-5 156 911 à groupement -COOH, résultant de la copolymérisation d'acide acrylique et d'alkyl(méth)acrylate en C₅ à C₁₆ de température de fusion allant de 20°C à 35°C et plus particulièrement de la copolymérisation :
   - d'acide acrylique, d'hexadécylacrylate et d'isodécylacrylate dans un rapport 1/16/3,
   - d'acide acrylique et de pentadécylacrylate dans un rapport 1/19,
   - d'acide acrylique, d'hexadécylacrylate, éthylacrylate dans un rapport 2,5/76,5/20,
   - d'acide acrylique, d'hexadécylacrylate et de méthylacrylate dans un rapport 5/85/10,
   - d'acide acrylique, de polyoctadécylméthacrylate dans un rapport 2,5/97,5.

   On peut aussi utiliser le polymère Structure « O » de National Starch tel que celui décrit dans le document US-A-5 736 125 de température de fusion de 44°C ainsi que les polymères semi-cristallins à chaînes pendantes cristallisables comportant des groupements fluorés tels que décrits dans les exemples 1, 4, 6, 7 et 8 du document WO-A-01/19333.
   On peut encore utiliser les polymères semi-cristallins de bas point de fusion obtenus par copolymérisation d'acrylate de stéaryle et d'acide acrylique ou de NVP tels que décrits dans le document US-A-5 519 063 ou EP-A- 550 745 et plus spécialement ceux décrits dans les exemples 1 et 2, ci-après, de préparation de polymère.
ii) Les polymères semi-cristallins présentant un point de fusion supérieur ou égal à 50°C sont notamment l'Intelimer décrit dans la brochure "Intelimer® polymers", Landec IP22 (Rev. 4-97) de température de fusion de 56°C, qui est un produit visqueux à température ambiante, imperméable, non-collant.
   On peut aussi utiliser les polymères semi-cristallins obtenus par copolymérisation de l'acrylate de béhényle et de l'acide acrylique ou de NVP, tels que décrits dans les documents US-A-5 519 063 et EP-A- 055 0745 et plus spécialement ceux décrits dans les exemples 3 et 4, ci-après, de préparation de polymère.

De préférence, les polymères semi-cristallins à bas point de fusion et/ou ceux à haut point de fusion ne comportent pas de groupement carboxylique.

Selon l'invention et de façon avantageuse, le composé à haut point de fusion (cristallin ou semi-cristallin) et celui à bas point de fusion sont dans un rapport en poids allant de 10/90 à 90/10 et mieux de 40/60 à 60/40, de préférence encore dans un rapport pondéral proche de 50/50.

De façon avantageuse, le rapport pondéral de polymère semi-cristallin à structure organique par rapport à la phase grasse liquide est de 0,20 à 0,60 et mieux de 0,25 à 0,50, obtenir un stick dur qui se délite au contact de la peau ou des lèvres et en particulier de dureté allant de 100 à 350 gf.

La gélification de la phase grasse est modulable selon la nature du ou des polymères et leurs concentrations respectives et peut être telle que l'on obtienne une structure rigide sous forme d'un bâton ou d'un stick.

Le taux de chaque polymère est choisi selon la dureté de la composition désirée et en fonction de l'application particulière envisagée. Les quantités respectives de polymère peuvent être telles qu'elles permettent l'obtention d'un solide délitable, présentant en particulier une dureté allant de 100 à 350 gf. Cette dureté peut être mesurée par la méthode dite du "fil à couper le beurre", qui consiste à couper un bâton de rouge à lèvres de 12,7 mm de diamètre et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme-force) nécessaire pour couper un stick dans ces conditions.

Cette dureté est telle que la composition est autoportée et peut se déliter aisément pour former un dépôt satisfaisant sur la peau et les lèvres. En outre, avec cette dureté, la composition de l'invention sous forme coulée notamment en stick résiste bien aux chocs.

De préférence, la composition de l'invention se présente sous forme d'un stick solide, de dureté allant de 100 gf à 350 gf, mesurée selon la méthode du "fil à couper le beurre". Il est toutefois possible d'utiliser une quantité de polymère semi-cristallin telle que la composition soit sous forme de pâte souple applicable au doigt ou à l'aide d'un applicateur sur les matières kératiniques.

En pratique, la quantité totale de polymère(s) semi-cristallin(s) représente de 0,1 à 80 % du poids total de la composition et mieux de 0,5 à 40 % et encore mieux de 3 à 30 %. De préférence, il représente de 15 % à 25% en poids de la composition.

Les bâtons ou sticks selon l'invention permettent, après application, d'obtenir un dépôt brillant, homogène en couleur, non collant, de bonne couvrance (c'est-à-dire que la peau ou les lèvres n'apparaît pas sous le maquillage).

### L'huile volatile

Par "huile volatile", on entend tout milieu non aqueux susceptible de s'évaporer au contact de la peau en moins d'une heure, à température ambiante (25°C) et pression atmosphérique (760 mm de Hg).

Le ou les huiles cosmétiques volatiles, liquides à température ambiante, ont notamment une pression de vapeur, mesurée à température ambiante et pression atmosphérique, allant de 10⁻³ à 300mm de Hg (0,266 Pa à 40 000 Pa), de préférence de 0,02 mm à 300mm de Hg (2,66 Pa à 40 000 Pa) et mieux allant de 0,1 à 90 mm de Hg (13 Pa à 12 000 Pa)..

Selon l'invention, ces huiles volatiles facilitent, notamment, l'application de la composition sur la peau, les lèvres ou les phanères. Ces huiles peuvent être des huiles hydrocarbonées, des huiles siliconés comportant éventuellement des groupements alkyle ou alkoxy pendants ou en bout de chaîne siliconée ou un mélange de ces huiles.

De préférence, les huiles volatiles sont des huiles cosmétiques choisies parmi les huiles n'ayant pas de point éclair, les huiles ayant un point éclair allant de 40°C à 100°C, et leurs mélanges, en vue de faciliter leur mise en oeuvre. En outre, ils présentent avantageusement une température d'ébullition à pression atmosphérique inférieure à 220°C et mieux inférieure à 210°C, notamment allant de 110 à 210°C. De préférence, ces huiles volatiles ne sont pas des monoalcools à au moins 7 atomes de carbone.

Comme huile volatile utilisable dans l'invention, on peut citer les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclo-tétrasiloxane, le décaméthylcyclopentasiloxane, le dodécaméthylcyclohexasiloxane, l'heptaméthyl hexyltrisiloxane, l'heptaméthyloctyl trisiloxane, l'hexaméthyldisiloxane, l'octaméthyl trisiloxane, le décaméthyl tétrasiloxane, le dodécaméthyl pentasiloxane et leurs mélanges.

Comme autre huile volatile utilisable dans l'invention, on peut citer les huiles volatiles hydrocarbonées ayant de 8 à 16 atomes de carbone et leurs mélanges et notamment les alcanes ramifiés en C₈-C₁₆ comme les isoalcanes (appelés aussi isoparaffines) en C₈-C₁₆, l'isododécane, l'isodécane, l'isohexadécane et par exemple les huiles vendues sous les noms commerciaux d'Isopars' ou de Permetyls, les esters ramifiés en C₈-C₁₆ comme le néo pentanoate d'iso-hexyle et leurs mélanges.

De préférence, on utilise l'isododécane (Permetyls 99 A), les isoparaffines en C₈-C₁₆ comme l'Isopar L, E, G ou H, leurs mélanges, éventuellement associés au décaméthyl tétrasiloxane ou au cyclopentasiloxane.

On peut aussi utiliser des huiles volatiles fluorées.

Ces huiles volatiles représentent notamment de 5 à 97,5 % du poids total de la composition, et mieux de 10 à 75 %, de préférence entre 20 et 50% du poids total de la composition. De façon générale, la quantité de solvant volatil est utilisée en une quantité suffisante pour obtenir des propriétés de sans transfert. Cette quantité sera adaptée par l'homme du métier en fonction de l'intensité des propriétés de sans transfert recherchée.

L'huile volatile représente de préférence 20 à 50 % en poids de la composition, de préférence de 30 à 40%, de préférence 35% environ.

L'huile volatile représente de préférence 40 à 60 % de la phase grasse liquide, de préférence de 45 à 55%.

Le rapport pondéral d'huile volatile par rapport au polymère semi-cristallin est avantageusement compris entre 1 et 2,5, de préférence de 1,5 à 2.

### La phase grasse liquide

Par "phase grasse liquide", au sens de la demande, on entend une phase grasse liquide à température ambiante (25°C) et pression atmosphérique (760 mm de Hg), composée d'un ou plusieurs corps gras liquides à température ambiante, appelés aussi huiles, compatibles entre eux. Cette phase grasse est macroscopiquement homogène.

Avantageusement, la phase grasse liquide, structurée par les polymères semi-cristallins, constitue la phase continue de la composition. Cette phase grasse peut contenir une ou plusieurs huiles apolaires ou non ou un mélange d'huile(s) apolaire(s) et d'huile(s) polaire(s)ou un mélange d'huiles polaires, différentes de l'alcool aliphatique décrit précédemment.

Les huiles apolaires selon l'invention sont en particulier les huiles siliconées telles que les polydiméthylsiloxanes (PDMS), linéaires ou cycliques, liquides à température ambiante ; les polydiméthylsiloxanes comportant des groupements alkyle, alcoxy ou phényle, pendant et/ou en bout de chaîne siliconée, groupements ayant de 2 à 24 atomes de carbone, liquides à température ambiante ; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates, liquides ; les hydrocarbures ou fluorocarbures linéaires ou ramifiés d'origine synthétique ou minérale, liquides, comme les huiles de paraffine et leurs dérivés, la vaseline, les polydécènes, le polyisobutène hydrogéné tel que le Parléam® vendu par la société Nippon Oil Fats, le squalane ; leurs mélanges. De préférence, les huiles apolaires utilisées sont des huiles apolaires du type hydrocarboné, liquides, d'origine minérale ou synthétique, choisies notamment parmi l'huile de Parleam® (isoparaffine hydrogénée), les isoparaffines, le squalane et leurs mélanges.

Avantageusement, la phase grasse liquide contient au moins une huile polaire et au moins une huile peu polaire, comme l'isononyl isononanonoate.

En particulier, les huiles polaires de l'invention sont :
- les huiles végétales hydrocarbonées à forte teneur en triglycérides constitués d'esters d'acides gras (en C₈ à C₂₄) et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812, et 818 par la société Dynamit Nobel;
- les huiles de synthèse de formule R₅COOR₆ dans laquelle R₅ représente le reste d'un acide gras supérieur linéaire ou ramifié comportant de 7 à 40 atomes de carbone et R₆ représente une chaîne hydrocarbonée ramifiée contenant de 3 à 40 atomes de carbone comme par exemple l'huile de Purcellin (octanoate de cétostéaryle), l'isononanoate d'isononyle, le benzoate d'alcool en C₁₂ à C₁₅;
- les esters et les éthers de synthèse comme le myristate d'isopropyle, le palmitate d'éthyl 2-hexyle, des octanoates, décanoates ou ricinoléates d'alcools ou de polyalcools, les esters hydroxylés comme le lactate d'isostéaryle, le malate de di-isostéaryle ; et les esters du pentaérythritol ;
- les acides gras ayant de 12 à 22 atomes de carbone comme l'acide oléique, linoléique ou linolénique ;
- leurs mélanges.
   La phase grasse liquide représente, en pratique, de 5 à 99 % du poids total de la composition, de préférence de 20 à 80 %. Avantageusement, elle représente au moins 60 % du poids total de la composition.

### La matière colorante

Avantageusement, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 50 % (en matière sèche) du poids total de la composition, de préférence de 5 à 30 % (si présente).

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,01 à 6 % (si présents). Les colorants hydrosolubles sont par exemple le jus de betterave, le bleu de méthylène et peuvent représenter jusqu'à 6 % du poids total de la composition.

Les pigments peuvent être blancs ou colorés, minéraux et/ou organiques, enrobés ou non. On peut citer, parmi les pigments minéraux, le dioxyde de titane ou de zinc, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium (tels que le D & C red 27, 21, 7, D & C yellow 5, 6, le F D & C blue n°1). Les pigments peuvent représenter de 0 à 40 % (0,01 à 40 %) notamment de 0,5 à 35 % et mieux de 2 à 25 % du poids total de la composition (si présents).

Les pigments nacrés peuvent être choisis parmi les pigments nacrés blancs tels que le mica notamment recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 25 % (0,05 à 25 %) du poids total de la composition et mieux de 0,1 à 15 % (si présents).

Avantageusement, les pigments et les nacres sont introduits dans la composition sous forme de pâte pigmentaire.

Par "pâte pigmentaire", on entend au sens de l'invention une dispersion colloïdale concentrée de particules colorées enrobées ou non dans un milieu continu, éventuellement stabilisée à l'aide d'un agent dispersant.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition de maquillage de l'invention peut se présenter sous la forme d'un produit coloré en particulier de la peau, présentant éventuellement des propriétés de soin ou de traitement, et être en particulier un fond de teint, un blush, un fard à joues ou à paupières, un produit anti-ceme, un eye-liner, un produit de maquillage du corps ; de maquillage des lèvres comme un rouge à lèvres, un brillant à lèvres ou un crayon à lèvres présentant éventuellement des propriétés de soin ou de traitement ; de maquillage des phanères comme les ongles, les cils sous forme de mascara, les sourcils et les cheveux. Elle est de préférence anhydre ou sous forme coulée.

Bien entendu la composition de l'invention doit être cosmétiquement acceptable, à savoir contenir un milieu physiologiquement acceptable non toxique et susceptible d'être appliqué sur la peau, les phanères ou les lèvres du visage d'êtres humains. Par cosmétiquement acceptable, on entend au sens de l'invention une composition d'aspect, d'odeur, de toucher et éventuellement de goût agréables.

On peut aussi utiliser dans la composition de l'invention au moins une cire telle que celles utilisées jusqu'à ce jour en cosmétique.

Une cire, au sens de la présente invention, est un composé gras lipophile, solide à température ambiante (25°C) et pression atmosphérique (760 mm de Hg), à changement d'état solide/liquide réversible, ayant une température de fusion supérieure à 40°C et mieux supérieure à 50°C, pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. Cette recristallisation dans le mélange peut être responsable de la diminution de la brillance dudit mélange. Aussi, avantageusement, la composition contient peu ou pas de cires classiques et notamment moins de 10 %, en poids, de cire classique et mieux moins de 5% par rapport au poids total de la composition.

Les cires classiques, au sens de la demande, sont celles généralement utilisées dans les domaines cosmétique et dermatologique ; elles sont notamment d'origine naturelle comme la cire d'abeilles, de Candellila, d'Ouricoury, du Japon, de fibres de liège ou de canne à sucre, les cires de paraffine, de lignite, les cires microcristallines de point de fusion > 50°C, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène et les cires obtenues par synthèse de Fischer-Tropsch à point de fusion > 50°C, les esters d'acides gras et les glycérides concrets à 50°C, les cires de silicone comme les alkyle, alcoxy et/ou esters de poly(di)méthylsiloxane solide à 50°C.

Avantageusement, la composition de l'invention contient peu ou pas de charges "matifiantes" et en particulier moins de 5 % de charge matifiante. Ceci est notamment le cas lorsqu'on souhaite obtenir un dépôt brillant sur les matières kératiniques comme les lèvres, les cils et les cheveux. Pour un fond de teint, on peut au contraire utiliser ce type de charges. Une charge matifiante est en général une charge qui absorbe la sueur et/ou le sébum de la peau comme les silices, talcs, argiles, kaolins, poudres de polyamide (Nylon®).

La composition selon l'invention peut être fabriquée par les procédés connus, généralement utilisés dans le domaine cosmétique.

L'invention a encore pour objet un procédé cosmétique de soin, de maquillage ou de traitement des matières kératiniques des êtres humains et notamment de la peau, des lèvres du visage et des phanères des êtres humains, comprenant l'application sur les matières kératiniques de la composition notamment cosmétique telle que définie ci-dessus.

L'invention a encore pour objet l'utilisation d'une huile comprenant au moins deux fonctions ester, dans une composition de maquillage comprenant a) au moins une phase grasse liquide structurée par au moins un polymère semi-cristallin à structure organique dont la température de fusion est supérieure ou égale à 30°C, et b) une matière colorante, la phase grasse liquide, la matière colorante, l'huile volatile et le polymère formant un milieu physiologiquement acceptable.

L'invention a encore pour objet l'utilisation d'une quantité suffisante d'une huile volatile, dans une composition cosmétique contenant un milieu physiologiquement acceptable comprenant a) au moins une phase grasse liquide structurée par au moins un polymère semi-cristallin à structure organique, dont la température de fusion est supérieure ou égale à 30°C, et b) une matière colorante, comme agent non transfert de ladite composition, ladite composition déposant sur les matières kératiniques, et notamment les lèvres, un film brillant et confortable.

L'invention a encore pour objet l'utilisation d'une huile volatile dans une composition de maquillage contenant un milieu physiologiquement acceptable comprenant au moins une phase grasse liquide structurée par au moins un polymère semi-cristallin à structure organique, pour obtenir une composition cosmétique sans-transfert.

L'invention est illustrée plus en détail dans les exemples suivants. Les quantités sont données en pourcentage massique.

### I) Exemples de fabrication de polymères semi-cristallins

### Exemple 1 : Homopolymère de point de fusion de 48°C

Dans un réacteur d'1l muni d'une agitation centrale avec ancre, d'un réfrigérant et d'un thermomètre, on introduit 120g de Parléam que l'on chauffe de la température ambiante à 80°C en 45 min. A 80°C, on introduit en 2h le mélange C₁ suivant :
40g de cyclohexane + 4g de Trigonox 141 [ 2,5 bis (2-éthyl hexanoyl peroxy) - 2,5 - diméthyl hexane].
30 min après le début de la coulée du mélange C₁, on introduit en 1h30 le mélange C₂ constitué de :
200 g d'acrylate de stéaryle + 400 g de cyclohexane.
A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans le Parléam.

Sa masse moléculaire moyenne en poids est de l'ordre 20 000-30 000 et sa température de fusion T_{f} est de 48°C, mesurée par D.S.C.

### Exemple 2 : Copolymère de point de fusion de 48°C

On applique le même mode opératoire que dans l'exemple 1, sauf que l'on utilise un mélange de 10 g de N-Vinyl Pyrrolidone et de 190 g d'acrylate de stéaryle.

Le polymère obtenu est à 60% en poids en matière active dans le Parléam, sa masse moléculaire moyenne en poids est de 43 000-53 000 et sa T_{f} de 48°C.

### Exemple 3 : Homopolymère de point de fusion de 58°C

On applique le même mode opératoire que dans l'exemple 1, sauf que l'on utilise de l'acrylate de béhényle en lieu et place de l'acrylate de stéaryle. Le polymère obtenu est à 60% en poids en matière active dans le Parléam. Sa masse moléculaire moyenne en poids est de 17 000-27 000 et sa T_{f} de 58°C.

### Exemple 4 : Copolymère de point de fusion de 58°C

On applique le même mode opératoire que dans l'exemple 2, sauf que l'on utilise de l'acrylate de béhényle en lieu et place de l'acrylate de stéaryle. Le polymère obtenu est à 60% en poids en matière active dans le Parléam®. Sa masse moléculaire moyenne en poids est de 23 500-33 500 et sa T_{f} de 58°C.

### II) Exemples de composition

### Exemple 5

| | |
|---|---|
| Polymère de l'exemple 1 | 14% |
| Polymère de l'exemple 3 | 10 % |
| Pigments | 8,7% |
| Isoparaffine hydrogénée | 16,2 % |
| Solsperse 21000 | 2 % |
| isododécane | 30 % |
| Silicone phénylée | 16,2 % |
| Lanoline liquide | 3% |

Mode de réalisation.
- Dans un poêlon peser les polymères semi-cristallins, les pâteux
- Incorporer les huiles non - volatiles et la charge
- Mettre le poêlon à chauffer au bain d'huile à 100°C (petit poêlon) ou 125°C (grand poêlon), sous agitation magnétique.
- Laisser fondre le mélange. Lorsque celui-ci est fluide et homogène, rajouter le broyat pigmentaire.
- Laisser sous agitation magnétique pendant 40 min.
- Baisser la température à 80°C. Lorsque celle-ci est atteinte ajouter le solvants volatils. Laisser le mélange s'homogénéiser
   Couler dans le moule.

Cette formule a été évaluée par des femmes et positionnée par rapport à une composition de l'état de la technique qui a été commercialisée sous la marque Rouge Captif ®. Les deux formules testées sont réalisées dans la même teinte. Les résultats de l'évaluation mettent en évidence les meilleures propriétés de brillance à l'application et à 1h de la formule de l'invention qui comprend les polymères semi-cristallins. Cette brillance est associée à un effet non-transfert qui est nettement en faveur de la formule à base des polymères semi-cristallins.

## Revendications

1. Composition de maquillage comprenant a) au moins une phase grasse liquide structurée par au moins un mélange d'un polymère semi-cristallin à structure organique choisi parmi les polymères à bas point de fusion ayant une température de fusion supérieure ou égale à 30°C et inférieure à 50°C, et d'un polymère semi-cristallin à structure organique choisi parmi les polymères à haut point de fusion ayant une température de fusion au moins égale à 50°C, b) une matière colorante et c) une huile volatile, la phase grasse liquide, la matière colorante, l'huile volatile et le polymère formant un milieu physiologiquement acceptable.

2. Composition selon la revendication 1, **caractérisée en ce que** l'huile volatile a une température d'ébullition à pression atmosphérique inférieure à 220°C et/ou une pression de vapeur, mesurée à température ambiante et pression atmosphérique, allant de 0,266 Pa à 40 000 Pa, et/ou un point éclair allant de 40°C à 100°C.

3. Composition selon la revendication 1, **caractérisée en ce que** l'huile volatile est choisie parmi
- les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone, notamment l'octaméthylcyclo-tétrasiloxane, le décaméthylcyclopentasiloxane, le dodécaméthylcyclohexasiloxane, l'heptaméthyl hexyltrisiloxane, l'heptaméthyloctyl trisiloxane, l'hexaméthyldisiloxane, l'octaméthyl trisiloxane, le décaméthyl tétrasiloxane, le dodécaméthyl pentasiloxane et leurs mélanges.
- les huiles volatiles hydrocarbonées ayant de 8 à 16 atomes de carbone et leurs mélanges et notamment les alcanes ramifiés en C₈-C₁₆ comme les isoalcanes (appelés aussi isoparaffines) en C₈-C₁₆, l'isododécane, l'isodécane, l'isohexadécane, les esters ramifiés en C₈-C₁₆ comme le néo pentanoate d'iso-hexyle et leurs mélanges.

4. Composition selon l'une des revendications précédentes, **caractérisée en ce que** l'huile volatile représente 20 à 50 % , de préférence 30 à 40% en poids de la composition.

5. Composition selon l'une des revendications précédentes, dans laquelle l'huile volatile représente 40 à 60 %, de préférence de 45 à 55% en poids de la phase grasse liquide.

6. Composition selon l'une des revendications précédentes, **caractérisée en ce que** le rapport pondéral d'huile volatile par rapport au mélange de polymères semi-cristallins est avantageusement compris entre 1 et 2,5, de préférence de 1,5 à 2.

7. Composition selon l'une quelconque des revendications précédentes, dans laquelle les polymères semi-cristallins ont une masse moléculaire moyenne en poids allant de 5 000 à 1 000 000, de préférence de 10 000 à 800 000. préférentiellement de 15 000 à 500 000.

8. Composition selon l'une des revendications précédentes, **caractérisée en ce que** les polymères semi-cristaltins sont choisis parmi :
- les copolymères séquencés de polyoléfines à cristallisation contrôlée,
- les polycondensats volatiles aliphatiques ou aromatiques et les covolatiles aliphatiques/aromatiques,
- les homo- ou co-polymères portant au moins une chaîne latérale cristallisable,
- leurs mélanges.

9. Composition selon l'une des revendications précédentes, **caractérisée en ce que** les polymères semi-cristallins sont choisis parmi les homopolymères et copolymères comportant de 50 à 100 % en poids de motifs résultant de la polymérisation de un ou plusieurs monomères porteurs de chaîne(s) latérale(s) hydrophobe(s) cristallisable(s),

10. Composition selon l'une des revendications précédentes, **caractérisée en ce que** les polymères semi-cristallins sont choisis parmi les homopolymères et copolymères résultant de la polymérisation d'au moins un monomère à chaîne(s) cristallisable(s) de formule X : 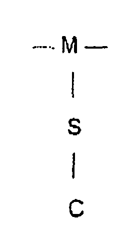 avec M représentant un atome du squelette polymérique
S représentant un espaceur
C représentant un groupe cristallisable
et leurs mélanges, avec « S-C » représentant une chaîne alkyle à au moins 11 atomes de carbone, éventuellement fluorée ou perfluorée.

11. Composition selon l'une des revendications précédentes, **caractérisée en ce que** les polymères semi-cristallins sont choisis parmi les polymères résultant de la polymérisation d'au moins un monomère choisi parmi l'acide acrylique, méthacrylique, crotonique, itaconique, maléique, l'anhydride maléique et leurs mélanges.

12. Composition selon l'une des revendications précédentes, **caractérisée en ce que** les polymères semi-cristallins sont choisis parmi les homopolymères et copolymères résultant de la polymérisation d'au moins un monomère à chaîne cristallisable choisis parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₁ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄ les alpha-oléfines en C₁₄ à C₂₄. les para-alkyl styrènes avec un groupe alkyle comportant de 12 à 24 atomes de carbone, leurs mélanges.

13. Composition selon l'une des revendications précédentes, **caractérisée en ce que** les polymères semi-cristallins sont des homopolymères d'alkyl(méth)acrylate ou d'alkyl(méth)acrylamide avec un groupe alkyle en C₁₄ à C₂₄, des copolymères de ces monomères avec un monomère hydrophile.

14. Composition selon l'une des revendications précédentes, **caractérisée en ce que** les polymères semi-cristallins sont des copolymères d'alkyl(méth)acrylate ou d'alkyl (méth)acrylamide avec un groupe alkyle en C₁₄ à C₂₄ avec un monomère de nature différente de l'acide (mèth)acrylique comme la N-vinylpyrolidone ou l'hydroxyéthyl (méth)acrylate, et leurs mélanges.

15. Composition selon l'une des revendications précédentes, **caractérisée en ce que** les polymères semi-cristallins représentent de 0,1 à 80 % du poids total de la composition et mieux de 0,5 à 40 % et encore mieux de 3 à 30 %, de préférence de 15 % à 25% en poids de la composition.

16. Composition selon l'une des revendications précédentes, **caractérisée en ce que** le polymère à haut point de fusion a une température de fusion Pf₁ telle que 55°C ≤ Pf₁ ≤ 150°C et de préférence 60°C ≤ Pf₁ ≤ 130°C.

17. Composition selon l'une des revendications précédentes, **caractérisée en ce que** le polymère ayant une température de fusion supérieure ou égale à 30°C et inférieure à 50°C, et le polymère ayant une température de fusion au moins égale à 50°C, sont présents dans un rapport en poids compris entre 90/10 et 10/90, de préférence entre 40/60 et 60/40, de préférence encore dans un rapport pondéral proche de 50/50.

18. Composition selon l'une des revendications précédentes, **caractérisée en ce que** la phase grasse contient au moins une huile polaire, et une huile peu polaire.

19. Composition selon l'une des revendications précédentes, **caractérisée en ce que** le rapport pondéral du mélange de polymères semi-cristallins et de la phase grasse liquide est compris de 0,20 à 0,60, de préférence de 0,25 à 0,50.

20. Composition selon l'une des revendications précédentes, **caractérisée en ce qu'**elle contient moins de 10 %, en poids, de cire et/ou moins de 5 % en poids de charge matifiante, par rapport au poids total de la composition.

21. Composition selon l'une des revendications précédentes, **caractérisée en ce que** la composition est sous forme anhydre.

22. Composition selon l'une quelconque des revendications précédentes, **caractérisée en ce qu'**elle se présente sous forme coulée.

23. Composition selon l'une quelconque des revendications précédentes, **caractérisée en ce qu'**elle se présente sous forme de mascara, d'eye-liner, de fond de teint, de rouge à lèvres, de déodorant, de produit de maquillage du corps, de fard à paupières ou à joues, de produit anti-cerne.

24. Composition selon la revendication 23, **caractérisée en ce qu'**elle se présente sous forme d'un stick solide, de dureté allant de 100 à 350 gf.

25. Rouge à lèvres comprenant a) au moins une phase grasse liquide structurée par au moins un polymère semi-cristallin à structure organique dont la température de fusion Pf₁ est telle que 55°C ≤ Pf₁ ≤ 150°C, et au moins un polymère semi-cristallin à structure organique dont la température de fusion Pf₂ est telle que 30°C ≤ Pf₂ < 50°C b) une matière colorante et c) une huile volatile.

26. Procédé cosmétique de maquillage comprenant l'application sur les matières kératiniques de la composition conforme à l'une des revendications 1 à 25.

27. Utilisation cosmétique d'une huile volatile dans une composition de maquillage contenant un milieu physiologiquement acceptable comprenant au moins une phase grasse liquide structurée par au moins un un mélange d'un polymère semi-cristallin à structure organique, choisi parmi les polymères a bas point de fusion ayant une température de fusion supérieure ou égale à 30°C et inférieur à 50°C, et d'un polymère semi-cristallin à structure organique choisi parmi les polymères a haut permet de fusion ayant une température de fusion au moins égale à 50°C pour obtenir une composition non-transfert dont le dépôt est brillant et confortable.

## Claims

1. Makeup composition comprising a) at least one liquid fatty phase structured with at least one mixture of a semi-crystalline polymer having an organic structure selected from low-melting polymers having a melting temperature of greater than or equal to 30°C and less than 50°C, and of a semi-crystalline polymer having an organic structure selected from high-melting polymers having a melting temperature of at least 50°C, b) a colorant and c) a volatile oil, the liquid fatty phase, colorant, volatile oil and polymer forming a physiologically acceptable medium.

2. Composition according to Claim 1, **characterized in that** the volatile oil has a boiling temperature at atmospheric pressure of less than 220°C and/or a vapour pressure, measured at ambient temperature and atmospheric pressure, ranging from 0.266 Pa to 40 000 Pa, and/or a flash point ranging from 40°C to 100°C.

3. Composition according to Claim 1, **characterized in that** the volatile oil is selected from
- linear or cyclic silicone oils having a viscosity at ambient temperature of less than 8 cSt and having in particular from 2 to 7 silicon atoms, these silicones optionally containing alkyl or alkoxy groups having from 1 to 10 carbon atoms, in particular octamethylcyclotetrasiloxane, decamethylcyclopentasiloxane, dodecamethylcyclo-hexasiloxane, heptamethylhexyltrisiloxane, heptamethyloctyltrisiloxane, hexamethyldisiloxane, octamethyltrisiloxane, decamethyltetrasiloxane, dodecamethyl-pentasiloxane and mixtures thereof,
- volatile hydrocarbon-based oils having from 8 to 16 carbon atoms and mixtures thereof, and in particular branched C₈-C₁₆ alkanes, for instance C₈-C₁₆ isoalkanes (also called isoparaffins), isododecane, isodecane, isohexadecane, branched C₈-C₁₆ esters such as isohexyl neopentanoate, and mixtures thereof.

4. Composition according to one of the preceding claims, **characterized in that** the volatile oil represents 20% to 50%, preferably 30% to 40% by weight of the composition.

5. Composition according to one of the preceding claims, wherein the volatile oil represents 40% to 60%, preferably from 45% to 55% by weight of the liquid fatty phase.

6. Composition according to one of the preceding claims, **characterized in that** the weight ratio of the volatile oil relative to the mixture of semi-crystalline polymers is advantageously between 1 and 2.5, preferably from 1.5 to 2.

7. Composition according to any one of the preceding claims, wherein the semi-crystalline polymers have a weight-average molecular mass ranging from 5 000 to 1 000 000, preferably from 10 000 to 800 000, preferentially from 15 000 to 500 000.

8. Composition according to one of the preceding claims, **characterized in that** the semi-crystalline polymers are selected from:
- block copolymers of polyolefins of controlled crystallization,
- aliphatic or aromatic volatile polycondensates and aliphatic/aromatic covolatiles,
- homopolymers or copolymers bearing at least one crystallizable side chain, and mixtures thereof.

9. Composition according to one of the preceding claims, **characterized in that** the semi-crystalline polymers are selected from homopolymers and copolymers comprising from 50% to 100% by weight of units resulting from the polymerization of one or more monomers bearing crystallizable hydrophobic side chain(s).

10. Composition according to one of the preceding claims, **characterized in that** the semi-crystalline polymers are selected from homopolymers and copolymers resulting from the polymerization of at least one monomer containing crystallizable chain(s), of formula X: 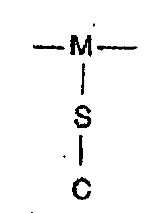 with M representing an atom of the
polymer skeleton,
S representing a spacer and
C representing a crystallizable group, and mixtures thereof, with "S-C" representing an alkyl chain having at least 11 carbon atoms which is optionally fluorinated or perfluorinated.

11. Composition according to one of the preceding claims, **characterized in that** the semi-crystalline polymers are selected from polymers resulting from the polymerization of at least one monomer selected from acrylic acid, methacrylic acid, crotonic acid, itaconic acid, maleic acid, maleic anhydride and mixtures thereof.

12. Composition according to one of the preceding claims, **characterized in that** the semi-crystalline polymers are selected from homopolymers and copolymers resulting from the polymerization of at least one monomer having a crystallizable chain, and selected from C₁₄-C₂₄ saturated alkyl (meth)acrylates, C₁₁-C₁₅ perfluoroalkyl (meth)acrylates, C₁₄ to C₂₄ N-alkyl(meth)acrylamides with or without a fluorine atom, vinyl esters containing C₁₄ to C₂₄ alkyl or perfluoroalkyl chains, vinyl ethers containing C₁₄ to C₂₄ alkyl or perfluoroalkyl chains, C₁₄ to C₂₄ alpha-olefins, para-alkylstyrenes with an alkyl group containing from 12 to 24 carbon atoms, and mixtures thereof.

13. Composition according to one of the preceding claims, **characterized in that** the semi-crystalline polymers are homopolymers of alkyl (meth)acrylate or of alkyl (meth) acrylamide with a C₁₄ to C₂₄ alkyl group and/or copolymers of these monomers with a hydrophilic monomer.

14. Composition according to one of the preceding claims, **characterized in that** the semi-crystalline polymers are copolymers of alkyl (meth)acrylate or of an alkyl(meth)acrylamide with a C₁₄ to C₂₄ alkyl group, with a monomer different in nature from (meth)acrylic acid, such as N-vinylpyrrolidone or hydroxyethyl (meth)acrylate, and mixtures thereof.

15. Composition according to one of the preceding claims, **characterized in that** the semi-crystalline polymers represent from 0.1% to 80% of the total weight of the composition and better still from 0.5% to 40%, and even better from 3% to 30%, preferably from 15% to 25%, by weight of the composition.

16. Composition according to one of the preceding claims, **characterized in that** the high-melting polymer has a melting temperature mp₁ such that 55°C ≤ mp₁ ≤ 150°C and preferably 60°C ≤ mp₁ ≤ 130°C.

17. Composition according to one of the preceding claims, **characterized in that** the polymer having a melting temperature of greater than or equal to 30°C and less than 50°C, and the polymer having a melting temperature of at least 50°C, are present in a ratio by weight of between 90/10 and 10/90, preferably between 40/60 and 60/40, more preferably in a weight ratio of close to 50/50.

18. Composition according to one of the preceding claims, **characterized in that** the fatty phase comprises at least one polar oil and one sparingly polar oil.

19. Composition according to one of the preceding claims, **characterized in that** the weight ratio of the mixture of semi-crystalline polymers and of the liquid fatty phase is from 0.20 to 0.60, preferably from 0.25 to 0.50.

20. Composition according to one of the preceding claims, **characterized in that** it contains less than 10% by weight of wax and/or less than 5% by weight of matting filler, relative to the total weight of the composition.

21. Composition according to one of the preceding claims, **characterized in that** the composition is in anhydrous form.

22. Composition according to any one of the preceding claims, **characterized in that** it is in cast form.

23. Composition according to any one of the preceding claims, **characterized in that** it is in the form of a mascara, eyeliner, foundation, lipstick, deodorant, body makeup product, eyeshadow or rouge or concealer product.

24. Composition according to Claim 23, **characterized in that** it is in the form of a solid stick with a hardness ranging from 100 to 350 gf.

25. Lipstick comprising a) at least one liquid fatty phase structured with at least one semi-crystalline polymer having an organic structure, the melting temperature mp₁ of which is such that 55°C ≤ mp₁ ≤ 150°C, and at least one semi-crystalline polymer having an organic structure, the melting temperature mp₂ of which is such that 30°C ≤ mp₂ < 50°C, b) a colorant, and c) a volatile oil.

26. Cosmetic makeup process comprising the application of the composition as set forth in one of Claims 1 to 25 to the keratin materials.

27. Cosmetic use of a volatile oil in a makeup composition containing a physiologically acceptable medium comprising at least one liquid fatty phase structured with at least one mixture of a semi-crystalline polymer having an organic structure selected from low-melting polymers having a melting temperature of greater than or equal to 30°C and less than 50°C, and of a semi-crystalline polymer having an organic structure selected from high-melting polymers having a melting temperature of at least 50°C, to obtain a non-transfer composition that forms a glossy and comfortable coating.

## Patentansprüche

1. Zusammensetzung zum Schminken, die a) mindestens eine flüssige Fettphase, die mit mindestens einem Gemisch aus einem halbkristallinen Polymer mit organischer Struktur, das unter den Polymeren mit niedrigem Schmelzpunkt ausgewählt ist, die eine Schmelztemperatur über 30 °C und unter 50 °C aufweisen, und einem halbkristallinen Polymer mit organischer Struktur strukturiert ist, das unter den Polymeren mit hohem Schmelzpunkt ausgewählt ist, die eine Schmelztemperatur von mindestens 50 °C aufweisen, b) ein Farbmittel und c) ein flüchtiges Öl enthält, wobei die flüssige Fettphase, das Farbmittel, das flüchtige Öl und das Polymer ein physiologisch annehmbares Medium bilden.

2. Zusammensetzung nach Anspruch 1, **dadurch gekennzeichnet, dass** das flüchtige Öl bei Atmosphärendruck eine Siedetemperatur unter 220 °C und/oder einen bei Raumtemperatur und Atmosphärendruck gemessenen Dampfdruck von 0,266 bis 40 000 Pa und/oder einen Flammpunkt von 40 bis 100 °C aufweist.

3. Zusammensetzung nach Anspruch 1, **dadurch gekennzeichnet, dass** das flüchtige Öl ausgewählt ist unter
- linearen oder cyclischen Siliconölen, die bei Raumtemperatur eine Viskosität unter 8 cSt aufweisen und insbesondere 2 bis 7 Siliciumatome besitzen, wobei diese Silicone gegebenenfalls Alkyl- oder Alkoxygruppen mit 1 bis 10 Kohlenstoffatomen aufweisen, insbesondere Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemischen,
- flüchtigen Kohlenwasserstoffölen mit 8 bis 16 Kohlenstoffatomen und deren Gemischen, insbesondere verzweigten C₈₋₁₆-Alkanen, wie Isoalkanen (die auch als Isoparaffine bezeichnet werden) mit 8 bis 16 Kohlenstoffatomen, Isododecan, Isodecan, isohexadecan, verzweigten Estern mit 8 bis 16 Kohlenstoffatomen, wie Isohexylneopentanoat und deren Gemischen.

4. Zusammensetzung nach einem der vorhergehenden Ansprüche, **dadurch gekennzeichnet, dass** das flüchtige Öl 20 bis 25 Gew.-% und vorzugsweise 30 bis 40 Gew.-% der Zusammensetzung ausmacht.

5. Zusammensetzung nach einem der vorhergehenden Ansprüche, wobei das flüchtige Öl 40 bis 60 Gew.-% und vorzugsweise 45 bis 55 Gew.-% der flüssigen Fettphase ausmacht.

6. Zusammensetzung nach einem der vorhergehenden Ansprüche, **dadurch gekennzeichnet, dass** das Gewichtsverhältnis des flüchtigen Öls und des Gemisches aus halbkristallinen Polymeren vorteilhaft im Bereich von 1 bis 2,5 und vorzugsweise 1,5 bis 2 liegt.

7. Zusammensetzung nach einem der vorhergehenden Ansprüche, **dadurch gekennzeichnet, dass** die halbkristallinen Polymere eine gewichtsmittlere Molmasse von 5 000 bis 1 000 000, vorzugsweise 10 000 bis 800 000 und bevorzugt 15 000 bis 500 000 aufweisen.

8. Zusammensetzung nach einem der vorhergehenden Ansprüche, **dadurch gekennzeichnet, dass** die halbkristallinen Polymere ausgewählt sind unter:
- Sequenzcopolymeren von Polyolefinen mit kontrollierter Kristallisation,
- aliphatischen oder aromatischen flüchtigen Polykondensaten und aliphatischen/aromatischen flüchtigen Coverbindungen,
- Homo- oder Copolymeren mit mindestens einer kristallisierbaren Seitenkette,
- deren Gemischen.

9. Zusammensetzung nach einem der vorhergehenden Ansprüche, **dadurch gekennzeichnet, dass** die halbkristallinen Polymere unter den Homopolymeren und Copolymeren mit 50 bis 100 Gew.-% Einheiten ausgewählt sind, die bei der Polymerisation eines oder mehrerer Monomere gebildet werden, die eine oder mehrere kristallisierbare hydrophobe Seitenkette(n) aufweisen,

10. Zusammensetzung nach einem der vorhergehenden Ansprüche, **dadurch gekennzeichnet, dass** die halbkristallinen Polymere unter den Homopolymeren und Copolymeren ausgewählt sind, die bei der Polymerisation mindestens eines Monomers mit kristallisierbarer (kristallisierbaren) Seitenkette(n) der Formel X gebildet werden: 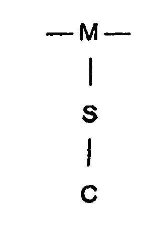 wobei M ein Atom des Polymergrundgerüsts ist,
S eine Spacergruppe bedeutet,
C eine kristallisierbare Gruppe ist und deren Gemischen, wobei «S-C» eine Alkylkette mit mindestens 11 Kohlenstoffatomen bedeutet, die gegebenenfalls fluoriert oder perfluoriert ist.

11. Zusammensetzung nach einem der vorhergehenden Ansprüche, **dadurch gekennzeichnet, dass** die halbkristallinen Polymere unter den Polymeren ausgewählt sind, die bei der Polymerisation mindestens eines Monomers gebildet werden, das unter Acrylsäure, Methacrylsäure, Crotonsäure, Itaconsäure, Maleinsäure, Maleinsäureanhydrid und deren Gemischen ausgewählt ist.

12. Zusammensetzung nach einem der vorhergehenden Ansprüche, **dadurch gekennzeichnet, dass** die halbkristallinen Polymere unter den Homopolymeren und Copolymeren ausgewählt sind, die bei der Polymerisation mindestens eines Monomers mit kristallisierbarer Kette gebildet werden, das unter den gesättigten C₁₄₋₂₄-Alkyl(meth)acrylaten, C₁₁₋₁₅-Perfluoralkyl(meth)acrylaten, C₁₄₋₂₄-N-Alkyl(meth)acrylamiden mit oder ohne Fluoratom, Vinylestern mit Alkyl- oder Perfluoralkylketten mit 14 bis 24 Kohlenstoffatomen, Vinylethern mit Alkyl- oder Perfluoralkylketten mit 14 bis 24 Kohlenstoffatomen, alpha-Olefinen mit 14 bis 24 Kohlenstoffatomen, para-Alkylstyrolen mit einer Alkylgruppe, die 12 bis 24 Kohlenstoffatome aufweist, und deren Gemischen ausgewählt ist,

13. Zusammensetzung nach einem der vorhergehenden Ansprüche, **dadurch gekennzeichnet, dass** die halbkristallinen Polymere Alkyl(meth)acrylat-Homopolymere oder Alkyl(meth)acrylamid-Homopolymere mit C₁₄₋₂₄-Alkylgruppe, Copolymere dieser Monomere mit einem hydrophilen Monomer sind.

14. Zusammensetzung nach einem der vorhergehenden Ansprüche, **dadurch gekennzeichnet, dass** die halbkristallinen Polymere Copolymere von Alkyl(meth)acrylat oder Alkyl(meth)acrylamid mit C₁₄₋₂₄-Alkylgruppe und einem Monomer sind, dessen Typ von (Meth)acrylsäure verschieden ist, wie N-Vinylpyrrolidon oder Hydroxyethyl(meth)actylat, und deren Gemische.

15. Zusammensetzung nach einem der vorhergehenden Ansprüche, **dadurch gekennzeichnet, dass** die halbkristallinen Polymere 0,1 bis 80 % des Gesamtgewichts der Zusammensetzung, besser 0,5 bis 40 % und noch besser 3 bis 30 %, vorzugsweise 15 bis 25 Gew.-% der Zusammensetzung ausmachen.

16. Zusammensetzung nach einem der vorhergehenden Ansprüche, **dadurch gekennzeichnet, dass** das Polymer mit hohem Schmelzpunkt eine Schmelztemperatur Pf₁ aufweist, die so ist, dass gilt: 55 °C ≤ Pf₁ ≤ 150 °C und vorzugsweise 60 °C ≤ Pf₁ ≤ 130 °C.

17. Zusammensetzung nach einem der vorhergehenden Ansprüche, **dadurch gekennzeichnet, dass** das Polymer, das eine Schmelztemperatur von mindestens 30 °C und unter 50 °C aufweist und das Polymer, das eine Schmelztemperatur von mindestens 50 °C aufweist, in einem Gewichtsverhältnis von 90/10 bis 10/90, vorzugsweise 40/60 bis 60/40 und noch bevorzugter in einem Gewichtsverhältnis in der Nähe von 50/50 enthalten sind.

18. Zusammensetzung nach einem der vorhergehenden Ansprüche, **dadurch gekennzeichnet, dass** die Fettphase mindestens ein polares Öl und ein wenig polares Öl enthält.

19. Zusammensetzung nach einem der vorhergehenden Ansprüche, **dadurch gekennzeichnet, dass** das Gewichtsverhältnis des Gemisches der halbkristallinen Polymere und der flüssigen Fettphase im Bereich von 0,20 bis 0,60 und vorzugsweise 0,25 bis 0,50 liegt.

20. Zusammensetzung nach einem der vorhergehenden Ansprüche, **dadurch gekennzeichnet, dass** sie weniger als 10 Gew.-% Wachs und/oder weniger als 5 Gew.-% mattierenden Füllstoff, bezogen auf das Gesamtgewicht der Zusammensetzung, enthält.

21. Zusammensetzung nach einem der vorhergehenden Ansprüche, **dadurch gekennzeichnet, dass** die Zusammensetzung in wasserireier Form vorliegt.

22. Zusammensetzung nach einem der vorhergehenden Ansprüche, **dadurch gekennzeichnet, dass** sie in gegossener Form vorliegt.

23. Zusammensetzung nach einem der vorhergehenden Ansprüche, **dadurch gekennzeichnet, dass** sie als Mascara, Eyeliner, Makeup, Lippenstift, Deodorant, Produkt zum Schminken des Körpers, Lidschatten, Wagenrouge oder Produkt gegen Augenringe vorliegt.

24. Zusammensetzung nach Anspruch 23, **dadurch gekennzeichnet, dass** sie als fester Stift mit einer Härte von 100 bis 350 gf vorliegt.

25. Lippenstift, der a) mindestens eine flüssige Fettphase, die mit mindestens einem halbkristallinen Polymer mit organischer Struktur, dessen Schmelztemperatur Pf₁ so ist, dass 55 °C ≤ Pf₁ ≤ 150°C, und mindestens einem halbkristallinen Polymer mit organischer Struktur strukturiert ist, dessen Schmelztemperatur Pf₂ so ist, dass 30 °C ≤ Pf₂ ≤ 50 °C, b) ein Farbmittel und c) ein flüchtiges Öl enthält.

26. Kosmetisches Verfahren zum Schminken, das das Aufbringen der Zusammensetzung nach einem der Ansprüche 1 bis 25 auf die Keratinsubstanzen umfasst.

27. Kosmetische Verwendung eines flüchtigen Öls in einer Zusammensetzung zum Schminken, die ein physiologisch akzeptables Medium aufweist, das mindestens eine flüssige Fettphase enthält, die mit mindestens einem Gemisch aus einem halbkristallinen Polymer mit organischer Struktur, das unter den Polymeren mit niedrigerem Schmelzpunkt, die eine Schmelztemperatur von mindestens 30 °C und unter 50 °C aufweisen, und einem halbkristallinen Polymer mit organischer Struktur strukturiert ist, das unter den Polymeren mit hohem Schmelzpunkt ausgewählt ist, die eine Schmelztemperatur von mindestens 50 °C aufweisen, um eine Zusammensetzung zu bilden, die sich nicht überträgt und deren Abscheidung glänzend und angenehm ist.
